(19)

Europäisches
Patentamt

European
Patent Office

Office européen
des brevets

(11) **EP 4 769 295 A1**

(12) **EUROPEAN PATENT APPLICATION**

(43) Date of publication:
**01.07.2026  Bulletin 2026/27**

(21) Application number: **25227437.8**

(22) Date of filing: **29.12.2025**

(51) International Patent Classification (IPC):
***G06T 5/60*** *(2024.01)*

(52) Cooperative Patent Classification (CPC):
**G06T 5/60;** G06T 2200/24; G06T 2207/20081;
G06T 2207/20084; G06T 2207/20092;
G06T 2207/30004

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **31.12.2024  US 202463740863 P**

(71) Applicant: **STRYKER CORPORATION
Portage, MI 49002-9711 (US)**

(72) Inventors:
• **LU, Xudong
  Portage, 49002 (US)**
• **RANGASAMY, Naveenkumar
  Portage, 49002 (US)**
• **VIVEKANANDAN, Vignesh
  Portage, 49002 (US)**
• **SCOTT, Ian Michael
  Portage, 49002 (US)**

(74) Representative: **V.O.
P.O. Box 87930
2508 DH Den Haag (NL)**

(54) **SYSTEMS, DEVICES, AND METHODS FOR MEDICAL IMAGE ENHANCEMENT USING MACHINE LEARNING**

(57)    A method for creating one or more enhanced medical image includes: receiving a user interaction via a user interface, the interaction comprising a user preference for image enhancement; adjusting a combined machine learning model comprising a combination of a plurality of specialized machine learning models based on the user interaction, wherein adjusting the combined machine learning model comprises adjusting a contribution of at least one specialized machine learning model of the plurality of specialized machine learning models to the combined model; creating one or more enhanced medical images by inputting medical image data into the combined machine learning model, wherein the combined machine learning model is configured to create one or more enhanced medical images from the medical image data; and displaying the one or more enhanced medical images.

**EP 4 769 295 A1**

**Description**

**CROSS-REFERENCE TO RELATED APPLICATION**

This application claims the benefit of U.S. Provisional Application No. 63/740,863, filed December 31, 2024, the entire contents of which is incorporated herein by reference.

**FIELD**

The present disclosure relates generally to medical image processing, and more specifically to medical image enhancement using machine learning.

**BACKGROUND**

Procurement of high-resolution images and video in medical settings enables medical professionals to accurately identify and treat certain anatomical features in the body. Medical imaging devices, however, often provide low-resolution, blurred, and noisy images. For instance, images obtained using flex-scopes, fiberscopes, or semirigid scopes are usually blurred and quite noisy in low resolution (e.g. 480p, 640p, etc.). Thus, image upscaling and enhancement techniques are frequently used to improve medical images to clearly depict anatomical features, which are often small and otherwise difficult to distinguish from their surrounding environment. Conventional image enhancement techniques, such as image interpolation, can improve image quality to some extent but often provide inadequate results relative to more advanced techniques, such as machine learning based enhancement. Machine learning models have been developed for image enhancement and provide better quality images over conventional enhancement techniques. However, such machine learning models are not adjustable to control image quality (e.g. smoothness, sharpness, contrast, etc.) in real-time. Thus, such models are not readily adapted to different user preferences or imaging conditions.

**SUMMARY**

Systems, devices, and methods disclosed herein combine a plurality of specialized machine learning models trained for different types of image enhancement into a single "combined" machine learning model. The combined machine learning model can be created by combining the learned parameters of a plurality of specialized machine learning models. The combined machine learning models disclosed herein may take in low-resolution medical images and generate "enhanced" (including both upscaling and enhancement, such as sharpening, brightening, etc.) medical images. The combined machine learning model can apply any number of different types and/or levels of image enhancement to the input images. The type or level of enhancement applied to the input medical images may be determined based on the weight assigned to the learned parameters of the respective specialized machine learning models used to create the combined machine learning model.

The combined machine learning models disclosed herein are also adjustable in real time by increasing or decreasing the contribution of one or more of the specialized machine learning models' parameters. Such adjustability is particularly advantageous for medical image enhancement. In medical imaging, the optimal combination of image enhancement techniques may vary for different imaging devices, surgical procedure type, anatomical feature being imaged, or even based on a given medical professional's preference. The systems disclosed herein enable on-the-fly adjustment of the combined machine learning model to perform any combination of image enhancement techniques. Thus, the combined machine learning model can be effectively tailored to many different imaging environments without requiring retraining of the specialized machine learning models or the combined machine learning model.

According to an aspect, an exemplary method for creating one or more enhanced medical images comprises: receiving a user interaction via a user interface, the interaction comprising a user preference for image enhancement; adjusting a combined machine learning model comprising a combination of a plurality of specialized machine learning models based on the user interaction, wherein adjusting the combined machine learning model comprises adjusting a contribution of at least one specialized machine learning model of the plurality of specialized machine learning models to the combined model; creating one or more enhanced medical images by inputting medical image data into the combined machine learning model, wherein the combined machine learning model is configured to create one or more enhanced medical images from the medical image data; and displaying the one or more enhanced medical images.

Optionally, adjusting the contribution of the at least one specialized machine learning model of the combination of specialized machine learning models comprises: adjusting a weight assigned to the at least one specialized machine learning model of the combination of specialized machine learning models; and generating a weighted combination of the plurality of specialized machine learning models including the at least one specialized machine learning model comprising the adjusted weight.

Optionally, generating the weighted combination of the plurality of specialized machine learning models comprises generating a weighted combination of corresponding learned parameters of each of the plurality of specialized machine learning models.

Optionally, each of the plurality of specialized machine learning models comprises an identical model architecture, and wherein: the combined machine learning model comprises the identical model architecture of the plurality of specialized machine learning models.

Optionally, the user interface comprises an interactive graphical user interface comprising one or more interactive affordances configured to receive the user preferences for image enhancement.

Optionally, the one or more interactive affordances comprises a slider, and wherein the user input causes a movement of a moveable element of the slider between a first position of the slider and a second position of the slider.

Optionally, adjusting the contribution of the at least one specialized machine learning models comprises: increasing, in real time, a contribution of a first specialized machine learning model based on the movement of the moveable element.

Optionally, adjusting the contribution of the at least one specialized machine learning models comprises: decreasing, in real time, a contribution of a second specialized machine learning model based on the movement of the moveable element

Optionally, adjusting the contribution of the at least one specialized machine learning models comprises: increasing, in real time, a contribution of a first specialized machine learning model based on the movement of the moveable element; and decreasing, in real time, a contribution of a second specialized machine learning model based on the movement of the moveable element

Optionally, the user interaction comprises a voice command.

Optionally, the method comprises: receiving a second user interaction via the user interface, the second user interaction comprising a user preference for image enhancement; adjusting the combined machine learning model based on the second user interaction; creating an updated enhanced medical image by inputting the medical image data into the combined machine learning model; and displaying the updated enhanced medical image

Optionally, the method comprises: receiving a second user interaction via the user interface, the second user interaction comprising a user preference for image enhancement; adjusting the combined machine learning model based on the second user interaction; creating a second enhanced medical image by inputting different medical image data into the combined machine learning model; and displaying the second enhanced medical image

Optionally, the combination of the plurality of specialized machine learning models comprises at least two specialized machine learning models selected from the group comprising: a machine learning model trained for upscaling image resolution and image contrast enhancement; a machine learning model trained for upscaling image resolution and image brightness enhancement; a machine learning model trained for upscaling image resolution and high dynamic range enhancement; a machine learning model trained for upscaling image resolution and image sharpness enhancement; a machine learning model trained for upscaling image resolution and image softness enhancement; and a machine learning model trained for upscaling image resolution and noise reduction

Optionally, each of the specialized machine learning models is a super-resolution convolutional neural network.

Optionally, adjusting the contribution of the at least one specialized machine learning model comprises increasing a contribution of a machine learning model trained for image contrast enhancement, and wherein creating the one or more enhanced medical images comprises generating an upscaled and contrast-enhanced medical image using the combined machine learning model.

Optionally, adjusting the contribution of the at least one specialized machine learning model comprises increasing a contribution of a machine learning model trained for image high dynamic range enhancement, and wherein creating the one or more enhanced medical images comprises generating an upscaled and high-dynamic-range-enhanced medical image using the combined machine learning model.

Optionally, adjusting the contribution of the at least one specialized machine learning model comprises increasing a contribution of a machine learning model trained for brightness enhancement, and wherein creating the one or more enhanced medical images comprises generating an upscaled and brightness-enhanced medical image using the combined machine learning model.

Optionally, adjusting the contribution of the at least one specialized machine learning model comprises increasing a contribution of a machine learning model trained for sharpness enhancement, and wherein creating the one or more enhanced medical images comprises generating an upscaled and sharpness-enhanced medical image using the combined machine learning model.

Optionally, adjusting the contribution of the at least one specialized machine learning model comprises increasing a contribution of a machine learning model trained for softness enhancement, and wherein creating the one or more enhanced medical images comprises generating an upscaled and softness-enhanced medical image using the combined machine learning model.

Optionally, adjusting the contribution of the at least one specialized machine learning model comprises increasing a contribution of a machine learning model trained for noise reduction, and wherein creating the one or more enhanced medical images comprises generating an upscaled and reduced-noise medical image using the combined machine learning model.

Optionally, the method includes receiving the medical image data from an endoscopic imaging device.

According to an aspect, an exemplary medical imaging system for creating an enhanced medical image comprises: one or more processors and a memory storing instructions, which, when executed by the one or more processors cause the system to: receive a user interaction via a user interface, the interaction comprising a user preference for image enhancement; adjust a combined machine learning model comprising a combination of a plurality of specialized machine learning models based on the user interaction, wherein adjusting the combined machine learning model comprises adjusting a contribution of at least one specialized machine learning model of the plurality of specialized machine learning models to the combined model; create one or more enhanced medical images by inputting medical image data into the combined machine learning model, wherein the combined machine learning model is configured to create one or more enhanced medical images from the medical image data; and display the one or more enhanced medical images.

Optionally, the system comprises one or more medical imaging devices configured to obtain the medical image data.

Optionally, the one or more medical imaging devices comprise at least one of an endoscopic imaging device a flex-scope imaging device, fiberscope imaging device, or semirigid scope imaging device, a pan-tilt-zoom (PTZ) camera, an open-field imaging device, and an in-light camera (ILC).

Optionally, the user interface comprises at least one of a interactive graphical user interface and an audio sensor.

Optionally, the plurality of specialized machine learning models comprise an identical model architecture.

Optionally, the plurality of specialized machine learning models have each been trained using a distinct discriminator model.

According to an aspect, an exemplary non-transitory computer-readable storage medium stores one or more programs for creating an enhanced medical image, the one or more programs comprising instructions, which when executed by one or more processors of an electronic device, cause the electronic device to: receive a user interaction via a user interface, the interaction comprising a user preference for image enhancement; adjust a combined machine learning model comprising a combination of a plurality of specialized machine learning models based on the user interaction, wherein adjusting the combined machine learning model comprises adjusting a contribution of at least one specialized machine learning model of the plurality of specialized machine learning models to the combined model; create one or more enhanced medical images by inputting medical image data into the combined machine learning model, wherein the combined machine learning model is configured to create one or more enhanced medical images from the medical image data; and display the one or more enhanced medical images.

According to an aspect, an exemplary method for enhancing a medical image comprises: assigning a weight to each of a plurality of specialized machine learning models; generating a combined machine learning model, comprising generating a weighted combination of the plurality of specialized machine learning models based on the weight assigned to each of a plurality of specialized machine learning models; creating one or more enhanced medical images by inputting medical image data into the combined machine learning model, wherein the combined machine learning model is configured to create one or more enhanced medical images from the medical image data.

Optionally, at least one of the plurality of specialized machine learning models is trained for a different type of image enhancement from a least one other specialized machine learning model of the plurality of specialized machine learning models.

Optionally, the method comprises causing display of the one or more enhanced medical images.

Optionally, the method comprises: receiving a user input, wherein the user input comprises an indication of a type of image enhancement; adjusting the combined machine learning model based on the user input, wherein adjusting the combined machine learning model comprises adjusting the weight assigned to at least one of the specialized machine learning models; creating a second enhanced medical image by inputting additional medical image data into the adjusted combined machine learning model; and causing display of the second enhanced medical image

Optionally, the weight is assigned to each of the plurality of specialized machine learning models based on a zoom setting of an imaging device that obtained the medical image data.

Optionally, the weight is assigned to each of the plurality of specialized machine learning models based on user preferences stored in association with a user profile.

Optionally, the weight is assigned to each of the plurality of specialized machine learning models based on any one or more of: a medical procedure, an imaging device, and an imaging modality.

According to an aspect, an exemplary medical imaging system for enhancing a medical image comprises one or more processors and a memory storing instructions, which, when executed by the one or more processors cause the system to: assign a weight to each of a plurality of specialized machine learning models; generate a combined machine learning model, comprising generating a weighted combination of the plurality of specialized machine learning models based on the weight assigned to each of a plurality of specialized machine learning models; create an enhanced medical image by inputting medical image data into the combined machine learning model, wherein the combined machine learning model is configured to create an enhanced medical image from the medical image data.

According to an aspect, an exemplary non-transitory computer-readable storage medium stores one or more programs for enhancing a medical image, the one or more programs comprising instructions, which when executed by one or more processors of an electronic device, cause the electronic device to: assign a weight to each of a plurality of specialized machine learning models; generate a combined machine learning model, comprising generating a weighted combination of the plurality of specialized machine learning models based on the weight assigned to each of a plurality of specialized machine learning models; create an enhanced medical image by inputting medical image data into the combined machine learning model, wherein the combined machine learning model is configured to create an enhanced medical image from the medical image data.

An exemplary method for creating a combined machine learning model for image enhancement comprises training a plurality of specialized machine learning models for image enhancement, wherein at least one of the plurality of specialized machine learning models is trained for a different type of image enhancement from at least one other of the plurality of specialized machine learning models; assigning a weight to each of the plurality of specialized machine learning models; and creating the combined machine learning model, comprising generating a weighted combination of the plurality of specialized machine learning models based on the weight assigned to each of a plurality of specialized machine learning models.

Optionally, at least one of the plurality of specialized machine learning models is trained using a different discriminator model than at least one other of the plurality of specialized machine learning models.

Optionally, the weight is assigned based on user preferences received based on a user input, the user input comprising an interaction with at least one of the one or more interactive affordances of a graphical user interface.

Optionally, generating the weighted combination of the plurality of specialized machine learning models comprises generating a weighted combination of corresponding learned parameters of the plurality of specialized machine learning models.

According to an aspect, an exemplary system for creating a combined machine learning model for image enhancement comprises one or more processors and a memory storing instructions, which, when executed by the one or more processors cause the system to: train a plurality of specialized machine learning models for respective types of image enhancement; assign a weight to each of the plurality of specialized machine learning models; and create the combined machine learning model, comprising generating a weighted combination of the plurality of specialized machine learning models based on the weight assigned to each of a plurality of specialized machine learning models.

According to an aspect, an exemplary non-transitory computer-readable storage medium stores one or more programs for creating a combined machine learning model for image enhancement, the one or more programs comprising instructions, which when executed by one or more processors of an electronic device, cause the electronic device to: train a plurality of specialized machine learning models for respective types of image enhancement; assign a weight to each of the plurality of specialized machine learning models; and create the combined machine learning model, comprising generating a weighted combination of the plurality of specialized machine learning models based on the weight assigned to each of a plurality of specialized machine learning models.

It will be appreciated that any of the variations, aspects, features and options described in view of the systems apply equally to the methods and vice versa. It will also be clear that any one or more of the above variations, aspects, features, and options can be combined.

## BRIEF DESCRIPTION OF THE FIGURES

The invention will now be described, by way of example only, with reference to the accompanying drawings, in which:

FIG. 1 shows an exemplary system for creating and using a combined machine learning model generated using multiple model interpolation according to some examples.

FIG. 2 shows a block diagram illustrating formation of a combined machine learning model that may be used for combined machine learning model of FIG. 1 according to some examples.

FIG. 3 shows a block diagram of an exemplary generative adversarial network for training one or more specialized machine learning models and combining the specialized machine learning models to create a combined machine learning model according to some examples.

FIG. 4 shows a block diagram of another exemplary generative adversarial network for training one or more specialized machine learning models and combining the specialized machine learning models to create a combined machine learning model according to some examples.

FIGS. 5A-5C show aspects of an interactive user interface for displaying enhanced medical images and adjusting the image enhancement in real time according to some examples.

FIG. 6 shows an exemplary method for creating one or more enhanced medical images according to some examples.

FIG. 7 shows another exemplary method for creating one or more enhanced medical images according to some examples.

FIG. 8 shows an exemplary computing device according to some examples.

## DETAILED DESCRIPTION

According to various aspects, systems and methods disclosed herein include creating one or more enhanced medical images using a combined machine learning model. As used herein, the term "enhanced" and its derivatives may refer to both upscaling and enhancing, and the terms "image(s)" and "image data" may encompass both single snapshot images and video frames. The combined machine learning model is created by generating a combination of specialized machine learning models trained for different types of image enhancement. During use, low-resolution medical images may be input into the combined machine learning model, and the combined machine learning model can apply a respective combination of upscaling and/or enhancement techniques to generate enhanced medical images optimized for different medical imaging environments. The enhanced images may be displayed to medical professionals during a medical procedure, augmenting human perception and enabling improved patient outcomes.

The combined machine learning models disclosed herein can be created by generating a weighted combination of corresponding learned parameters from a plurality of specialized machine learning models. Respective specialized machine learning models may be trained for a different type or level of image enhancement. For instance, each specialized machine learning model may be trained to upscale an input image, perform a particular type of image enhancement, such as contrast enhancement, brightness enhancement, smoothening, sharpening, etc., and/or both upscale the input image and perform a particular type of image enhancement. In some examples, each of a plurality of the specialized machine learning models may be trained to perform a different type of image enhancement. In some examples, a plurality of specialized machine learning models may be trained to perform different levels (e.g., different magnitudes/degrees) of the same type of image enhancement. The learned parameters of each of the specialized machine learning models may thus be different. However, the specialized machine learning models may share an identical model architecture. For instance, specialized machine learning models described herein may be super-resolution convolutional neural networks, sharing a convolutional neural network architecture. Corresponding parameters of the different specialized machine learning models can thus be combined using multiple-model interpolation to generate a combined model that shares the same model architecture.

Different medical imaging environments may call for different types of image enhancement. For example, in Ear, Nose, and Throat (ENT) surgeries, where the space is small and narrow, the deeper or posterior structure often appear darker than the anterior structures. In this environment, global contrast enhancement may be useful. During endourology procedures like ureteroscopy, cystoscopy, the images generally appear smoothened due to fluid medium and increased sharpness will be useful in this environment. In pelvic surgeries, preserving vital structures like ureter and hypogastric nerve plexus is important. In these situations, increased color contrast maybe helpful in identifying those vital structures. In surgeries like TURBT (Trans urethral resection of bladder tumor) with special imaging modes, increased contrast will be useful in differentiating cancerous lesions. During laparoscopic procedures with cautery tools, dehazing can help refine details, but without smoke, the same dehazing amplifies noise and over-sharpens the image, making it look cartoonish.

The combined machine learning models disclosed herein can be adjusted in real time, for instance, to increase contrast, brightness, smoothing, sharpening, etc. Adjusting the combined machine learning model may include adjusting the type of image enhancement performed by the combined machine learning model. In some examples, the level of a given type of image enhancement can be adjusted (e.g. by increasing or decreasing an amount of super-resolution upscaling, etc.) without necessarily combining (or adjusting a combination of) specialized models trained for different types of enhancement. Thus, the systems and methods disclosed herein may enable real-time adjustment of the level of one type of image enhancement (e.g., image resolution upscaling or contrast enhancement), without necessarily blending it with other types of image enhancement. The combined machine learning model can be adjusted by changing the contribution of one or more of the specialized machine learning models to the combined machine learning model. To enable real-time adjustment of the combined machine learning model, the learned parameters of the respective specialized machine learning models may be assigned different weights, and thus may contribute relatively more or less to the combined machine learning model. The weights can be adjusted in real time based on user interactions with the system and based on detected changes in imaging conditions. Thus, the system is highly adaptable to different environments and to the preferences of different users.

Moreover, the systems and methods disclosed herein provide technical advantages over existing image enhancement systems. The systems and methods disclosed herein provide improved image quality over conventional image enhancement techniques, enable real-time adjustment based on user preferences and imaging conditions, and do not rely on blending results of multiple super-resolution machine learning models executed in parallel. Conventional image enhancement techniques using machine learning models (e.g., super-resolution deep learning models) lack the control to adjust the desired image quality (e.g. smoothness, sharpness, contrast, etc.) in real time. Further, running multiple super-resolution deep learning models in parallel and blending their results is computationally expensive. Significant computational resource efficiencies are achieved by instead combining multiple specialized machine learning models into a single combined machine learning model, as disclosed herein.

It will be appreciated that any of the variations, aspects, features, and options described in view of the systems apply equally to the methods and vice versa. It will also be clear that any one or more of the above variations, aspects, features, and options can be combined. Also, it should be appreciated that various features of the systems and methods disclosed herein would be advantageous outside of medical imaging.

In the following description of the various examples, it is to be understood that the singular forms "a," "an," and "the" used in the following description are intended to include the plural forms as well, unless the context clearly indicates otherwise. It is also to be understood that the term "and/or" as used herein refers to and encompasses any and all possible combinations of one or more of the associated listed items. It is further to be understood that the terms "includes," "including," "comprises," and/or "comprising," when used herein, specify the presence of stated features, integers, steps, operations, elements, components, and/or units but do not preclude the presence or addition of one or more other features, integers, steps, operations, elements, components, units, and/or groups thereof.

### *System for Image Enhancement*

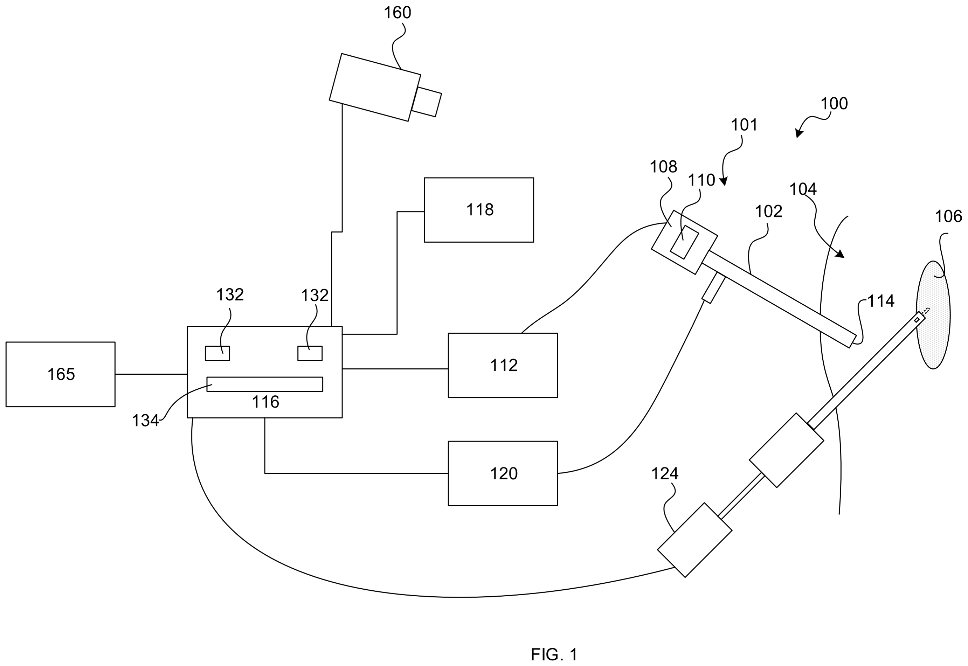

FIG. 1 illustrates an exemplary computing system 100 for creating enhanced medical images. Computing system 100 may include a medical data processing device 116 configured to process medical image data obtained using an imaging device, such as imaging device 101 and/or imaging device 160. Medical image data may be acquired during a medical procedure using imaging device 101 and/or imaging device 160 and may be transmitted to the medical data processing device 116. The medical data processing device 116 may generate enhanced medical images by inputting the medical image data into one or more machine learning models configured to generate enhanced images.

The medical data processing device 116 may generate and/or store a combined machine learning model 134 configured to generate enhanced medical images. The combined machine learning model 134 may be created (e.g., by medical data processing device 116) by combining a plurality of specialized machine learning models 132. The plurality of specialized machine learning models 132 have the same respective model architecture (e.g., a convolutional neural network (CNN) architecture), but each may be trained for a different type of image enhancement (e.g., image upscaling, brightness enhancement, sharpness enhancement). The system 100 may generate a weighted combination of the specialized machine learning models 132 by combining corresponding learned parameters from each of the specialized machine learning models 132 to create the combined machine learning model 134. The weight assigned to the parameters of the respective specialized machine learning models 132 may define their contribution to the combined machine learning model 134. In some examples, the medical data processing device 116 may use medical image data obtained using one or more imaging devices (e.g., imaging device 101 and/or imaging device 160) to train the plurality of specialized machine learning models 132. In some examples, the plurality of specialized machine learning models 132 are trained using a different computing system and stored in medical data processing device 116.

In some examples, a default contribution is assigned to the respective specialized machine learning models 132 to create a default combined machine learning model 134. The default contribution of different specialized machine learning models 132 to the combined machine learning model 134 may be assigned based upon an imaging modality, type of imaging device, resolution of an imaging device, type of surgical procedure, and/or stored user preferences in a user profile of a surgeon or other medical professional. Different default combined machine learning models 134 may be created and stored in medical data processing device 116 (and/or at a remote server, on the cloud, etc.) and may be activated based on a user selection, a type of imaging device being used, a medical procedure in which medical data processing device 116 is being used, etc.

The combined machine learning model 134 may be adjustable in real-time for different types of image enhancement by increasing and/or decreasing weights (in the weighted combination) assigned to the learned parameters of respective specialized machine learning models 132. In some examples, the combined machine learning model 134 is adjustable based on user inputs (e.g., indicating image enhancement preferences, such as a preferred type of enhancement). Medical data processing device 116 may also automatically adjust the combined machine learning model 134 based on detected changes in imaging conditions. For instance, the medical data processing device 116 may detect image data from a different imaging device, a change in lighting, a change in zoom settings, etc. and adjust the combined machine learning model 134 to increase and/or decrease a type of image enhancement (by changing weights assigned to the specialized machine learning models) to optimize the combined machine learning model 134 for the new imaging conditions.

Imaging device 101 may be an endoscopic imager and may include a camera head 108 mounted to an endoscope 102. The endoscope 102 can be configured for insertion into a surgical cavity 104 for imaging tissue 106 within the surgical cavity 104 during a medical procedure. The endoscopic camera head 108 includes one or more imaging sensors 110. Light generated by a light source 120 may be directed through the endoscope 102 to the surgical cavity 104. Light reflected by and/or emitted from the tissue 106 (such as fluorescence light emitted from fluorescing targets that are excited by fluorescence excitation illumination light provided by the light source 120) is received at the distal end 114 of the endoscope 102. The light is propagated by the endoscope 102, such as via one or more optical components (for example, one or more lenses, prisms, light pipes, or other optical components), to the camera head 108, where it is directed onto the one or more imaging sensors 110. One or more filters (not shown) may be included in the endoscope 102, in a coupler (not shown) connecting the endoscope 102 to the camera head 108, and/or in the camera head 108 for filtering a portion of the light received from the tissue 106 (such as fluorescence excitation light). In some examples, imaging device 101 may alternatively be a flex-scope, fiberscope, or semirigid scope. The imaging device 160 may be a pan-tilt-zoom (PTZ) camera in an operating room, an open-field imaging device, an in-light camera (ILC), etc.

The one or more imaging sensors 110 generate pixel data that can be transmitted to a camera control unit 112 that is communicatively connected to the camera head 108. The camera control unit 112 can generate endoscopic images (as used herein, "endoscopic image(s)" encompasses single snapshot images and/or a sequence of video frames) from the pixel data that shows the tissue being viewed by the endoscopic imager 101. The endoscopic images obtained using imaging device 101 and/or images obtained using imaging device 160 can be transmitted to medical data processing device 116 for further image processing, storage, display, and/or routing to an external device (not shown). The medical data processing device 116 receives the endoscopic images from the camera control unit 112. The endoscopic images can be transmitted to one or more displays 118, from the camera control unit 112 and/or the medical data processing device 116, for visualization by medical personnel, such as by a surgeon for visualizing the surgical cavity 104 during a surgical procedure on a patient. The camera control unit 112 and/or the medical data processing device 116 may be configured to send control signals to the light source 120 and/or the camera head 108 to control one or more aspects of the imaging, such as a timing sequence of light provided by the light source 120 (e.g., a sequence of white light and fluorescence excitation light), an amount of light provided by the light source 120, and/or a gain of the one or more imaging sensors 110.

One or more surgical tools 124 may be used in the surgical cavity 104 to manipulate tissue 106 during a surgical procedure on the patient, and the surgical tools may be captured in the images captured by the camera head 108 and displayed on the display 118 so that the medical personnel can view the interaction between the one or more surgical tools and the tissue. As described further below, the system 100 may analyze endoscopic image data using one or more machine learning models (e.g., combined machine learning model 134) and may generate and display visualizations (e.g., on display 118) based on the endoscopic data.

### Creating a Combined Machine Learning Model

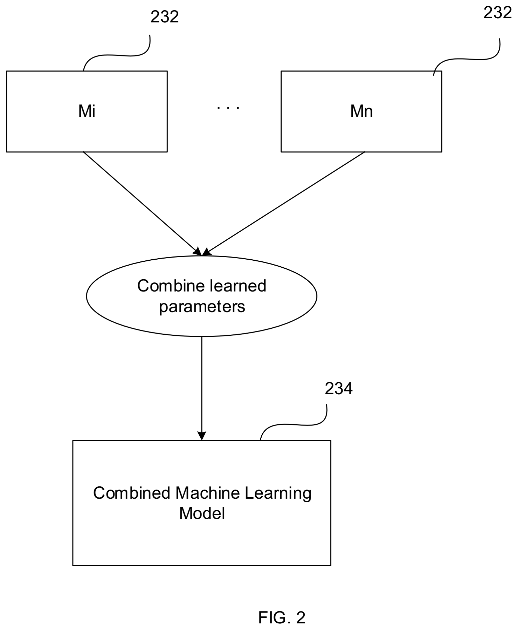

FIG. 2 shows a block diagram illustrating formation of a combined machine learning model 234 that may be used for combined machine learning model 134 of FIG. 1. Combined machine learning model 234 is created by combining a plurality of specialized machine learning models 232. Each of the plurality of specialized machine learning models 232 may have the same model architecture but may be trained for a different respective type or level of image enhancement. A different type of image degradation, a different discriminator model, or both may be used during training of each of the specialized machine learning models 232 to finetune each of the specialized machine learning models 232 for a respective type of enhancement.

For example, each of specialized machine learning models 232 may have a convolutional neural network (CNN) model architecture. A first specialized machine learning model 232 (e.g., $M_1$) may be trained for a first type or level of image enhancement (e.g., upscaling image resolution and enhancing contrast). A second specialized machine learning model 232 (e.g., $M_i$) may be trained for a second type or level of image enhancement (e.g., upscaling image resolution and image softness enhancement). A third specialized machine learning model 232 (e.g., $M_n$) may be trained for a third type or level of image enhancement (e.g., upscaling image resolution and high dynamic range enhancement). The learned parameters of the respective specialized machine learning models 232 can then be combined to create combined machine learning model 234. For instance, the specialized machine learning models 232 may be combined according to the following equation:

$$Combined\ Machine\ Learning\ Model = \sum_{i=1}^{N} \alpha_i M_i\ ,\ for\ \alpha_i \in [0,1]\ and\ \sum_{i=1}^{N} \alpha_i = 1$$

where $Mi$ corresponds to the learned parameters of the respective specialized machine learning models 232 (e.g., specialized machine learning models $i$ through $N$), and $\alpha_i$ corresponds to the weight assigned to the learned parameters of each respective specialized machine learning model 232. The $\alpha_i$ weights can be tuned (e.g., based on user inputs, automatically based on detected imaging conditions, etc.) to change the contribution of the learned parameters of the respective specialized machine learning models 232 (e.g. more smoothness). In some examples, the weight assigned to each model in the combination is the same. In some examples, the weight assigned to each model in the combination is different, emphasizing a particular type of image enhancement over others. In some examples, only one specialized machine learning model is assigned a non-zero weight. In some examples, all specialized machine learning models are assigned a non-zero weight. Accordingly, the combined machine learning model 234 is adjustable for different types or levels of enhancement.

In some examples, the weight assigned to the respective specialized machine learning models may be based on a surgeon performing a procedure, a type of procedure, a type of imaging device being used, a configuration of the imaging device being used (e.g., a zoom setting) an imaging modality, etc. Combined machine learning models can thus be configured according to default settings for predefined imaging conditions, such as those listed above. The defaults can then be adjusted (e.g., by users or automatically by the system) according to changes in imaging conditions and/or based on user preferences.

While two specialized machine learning models 232 are shown in FIG. 2, it should be understood that any number of specialized machine learning models 232 may be trained and combined to form the combined machine learning model 234. Additionally, it should be understood that a CNN model architecture is only one example, and the specialized machine learning models disclosed herein may share other architectures, including: Recurrent Neural Networks (RNN), various different types of CNN architectures (e.g., UNET, ResNet), Generative Adversarial Networks (GAN), Diffusion models, Transformer Models, etc. The aforementioned model architectures are meant to be exemplary and are in no way limiting.

### *Training Specialized Machine Learning Models*

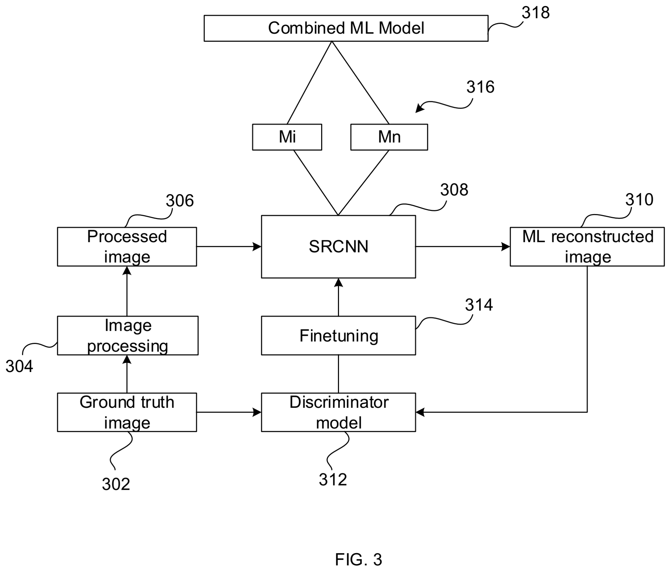

FIG. 3 shows a block diagram of an exemplary generative adversarial network for training one or more specialized machine learning models (e.g., specialized machine learning models 132, 232) and combining the specialized machine learning models to create a combined machine learning model (e.g., combined machine learning model 134, 234). The specialized machine learning models may be or include generator models, such as a super-resolution convolutional neural network (SRCNN) model. In some examples, the same discriminator may be used for training the different specialized machine learning models, but different image degradation methods may be used to train the specialized machine learning models for respective types or levels of enhancement. In some examples, a different discriminator model is used for training one or more of the specialized machine learning models based on different feature extraction and cost functions for the same degraded data-in such examples, the penalty from different cost functions may be different for the same image degradation, and thus the enhancement effect may be different. Thus, each specialized machine learning model may have the same model architecture, as described with reference to FIGS. 1 and 2, but may be trained using a distinct discriminator model and/or distinct training data to achieve a different type or level of image enhancement. Any or all aspects of the process described with reference to FIG. 3 may be performed using one or more components of system 100 described above. Any or all aspects of the process described with reference to FIG. 3 may alternatively be performed using a different computing system configured to train a plurality of specialized machine learning models. Further, it should be understood that, while described with reference to an image, the process described below could equally apply to a plurality of images and/or a video segment.

At block 302, a ground truth high-resolution (e.g., 1080p, 4k, etc.) image may be obtained. The ground truth image may be obtained using a high-resolution (e.g., 1080p, 4k, etc.) imaging device. The ground truth image may be a medical image obtained during a medical procedure. The medical image may depict one or more anatomical structures. Use of medical images as training data for the specialized machine learning models may enable the machine learning models described herein to learn features particularly important for medical image enhancement. For example, vessels in medical images may be or include fine structures. A machine learning model trained for image enhancement using non-medical image data may tend to smoothen out such fine structures because fine texture information is often treated as noise. By using high-quality ground truth medical images for training, models may learn to discriminate these fine textures as features to enhance rather than noise to smooth. The ground truth images used to train the machine learning models described herein may additionally, or alternatively, be obtained from any public or private data source. At block 304, the ground truth image may be processed using one or more image processing techniques. The ground truth image may be subject to one or more downscaling and image degradation (e.g., introduction of noise, blur, etc.) techniques to obtain a processed image 306.

At block 308, the processed image may be input into a machine learning model configured to reconstruct the ground truth image based on the processed image. The machine learning model used at block 308 may be an SRCNN model, such as the SRResNet model described in Christian Ledig, et al., Photo-Realistic Single Image Super-Resolution Using a Generative Adversarial Network, CVPR (2017) https://doi.org/10.48550/arXiv.1609.04802, which is incorporated herein by reference in its entirety. The machine learning model may output a reconstructed image at block 310. The reconstructed image and the ground truth image may be input into a discriminator model at block 312. The discriminator model may be configured to distinguish between the ground truth image and the reconstructed image. The discriminator may be a CNN classifier (such as the VGG16 model described in Photo-Realistic Single Image Super-Resolution Using a Generative Adversarial Network, CVPR (2017), https://doi.org/10.48550/arXiv.1609.04802), which takes the reconstructed image and ground truth high-resolution image and classifies them as real high-resolution images or fake images.

The machine learning model used at block 308 can be finetuned at block 314 based on the output of the discriminator model to reconstruct the ground truth image data more accurately and/or train the specialized machine learning model for a given image enhancement type. At block 316, a trained specialized machine learning model is obtained. The training process is an iterative process between generator and discriminator. The loss of the discriminator may be combined with content loss between the reconstructed/generated high-resolution image and the ground truth high-resolution image to finetune the generator. A plurality of specialized machine learning models (e.g., model $M_i$, model $M_n$) may be trained according to blocks 302 through 314. At block 318, the corresponding parameters of each specialized machine learning model trained according to blocks 302 through 314 may be combined to form a combined machine learning model (e.g., combined machine learning model 134, 234).

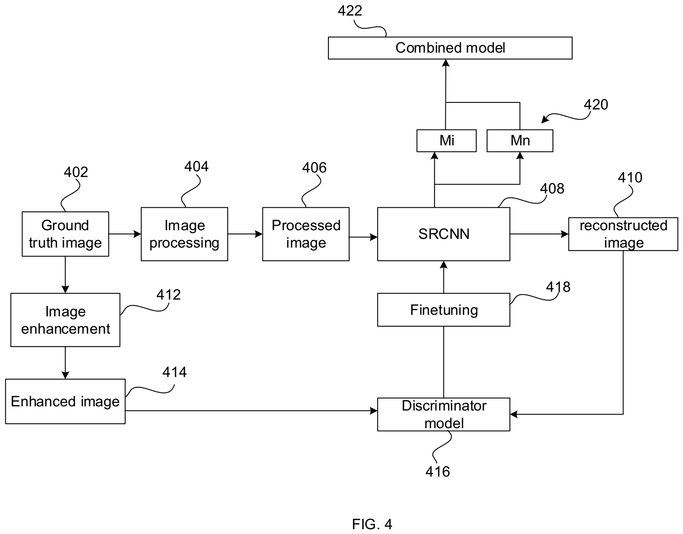

FIG. 4 shows a block diagram of another exemplary generative adversarial network for training one or more specialized machine learning models. The training process shown in FIG. 4 includes steps for enhancing ground truth images to reinforce the enhancement of different discriminators. To reinforce the enhancement of different discriminators, the ground truth image can first be enhanced (e.g., using conventional image processing techniques) for contrast enhancement, sharpness enhancement, and/or high dynamic range (HDR) enhancement. Any or all aspects of the process described with reference to FIG. 3 may be performed using one or more components of system 100 described above. Any or all aspects of the process described with reference to FIG. 3 may alternatively be performed using a different computing system configured to train a plurality of specialized machine learning models. Further, it should be understood that, while described with reference to an image, the process described below could equally apply to a plurality of images and or a video segment.

At block 402 a high-resolution (e.g., 1080p or 4k) ground truth image is obtained, for instance, using a high-resolution medical imaging device or from a public or private database that stores high-resolution images. The ground truth image may be a medical image obtained during a medical procedure. The medical image may depict one or more anatomical structures. At block 404, the ground truth image may be processed using one or more image processing techniques. The ground truth image may be subject to one or more downscaling and image degradation (e.g., introduction of noise, blur, etc.) techniques to obtain a processed (e.g., downscaled and degraded) image 406. At block 408, the processed image may be input into a machine learning model configured to generate a reconstructed image 410 based on the processed image 406. The machine learning model used at block 408 may be a SRCNN model, such as the one described with reference to FIG. 3.

At block 412, the ground truth image may be subjected to one or more image enhancement techniques to generate an enhanced image 414. At block 416, the enhanced image 414 and the reconstructed image 410 may be input into discriminator model at block 416. The discriminator model may be a CNN classifier, such as the VGG16 model described with reference to FIG. 3. The discriminator model used at block 416 may be configured to take the reconstructed image 410 and enhanced image 414 and classify them as real or fake. The machine learning model used at block 408 can be finetuned at block 418 based on the output of the discriminator model to reconstruct the enhanced image data more accurately and/or train the specialized machine learning model for a given image enhancement type. The exemplary training process shown in FIG. 4 may thus be used to train specialized machine learning models for image enhancement methods such as contrast enhancement, sharpness enhancement, and/or high dynamic range (HDR) enhancement.

At block 420, a trained specialized machine learning model is obtained. The training process is an iterative process between generator and discriminator. The loss of the discriminator may be combined with content loss between generated/reconstructed image 410 and enhanced image 414 to finetune the generator machine learning model used at block 408. A plurality of specialized machine learning models (e.g., model $M_i$, model $M_n$) may be trained according to blocks 402 through 418. At block 420, the corresponding parameters of each specialized machine learning model trained according to blocks 402 through 418 may be combined to form a combined machine learning model (e.g., combined machine learning model 134, 234).

### User Interfaces

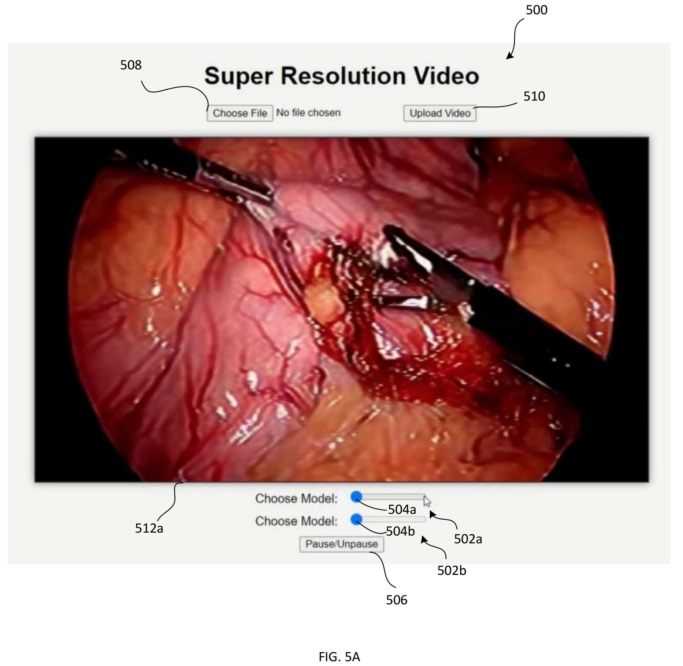

FIGS. 5A-5C illustrate aspects of an interactive user interface 500 for displaying enhanced medical images and adjusting the image enhancement in real time. Interactive user interface 500 may include or be displayed on display 118 of system 100. The interactive user interface 500 is configured to display images (including video) obtained during a medical procedure. In some examples, the interactive user interface 500 may display non-enhanced medical images in a default mode. Initializing the system in a default mode in which no image enhancement is applied may be advantageous for several reasons. For instance, a trained medical professional might know what should be visible in an image (e.g., a given structure, organ, etc.) and if it is missing from the image, the medical professional may determine that a type of image enhancement is needed to render the object visible. Displaying an unenhanced image in a default mode may provide a user with insight into the specific type of enhancement that may be beneficial. For instance, if it appears that an object may be blending into its surroundings, the user might activate image enhancement, thus activating a combined machine learning model, and may increase sharpness and/or contrast by adjusting the combined machine learning model as described herein. The interactive user interface 500 may enable a user to activate image enhancement mode via a voice command (e.g., using an audio sensor), using an affordance included on the interactive interface, or other mechanism. Upon activation of the image enhancement mode, the combined machine learning model (e.g., as described throughout) may generate an enhanced medical image that is displayed on a screen of the interactive user interface 500. The combined machine learning model may also be initialized in a default mode (e.g., based on the medical procedure type, imaging device, medical professional, etc.), and the user can use the interactive interface to adjust the combined machine learning model.

User interface 500 includes a plurality of interactive affordances and may include, for example, affordance 508 enabling a user to select an image or video file, affordance 510 enabling users to upload an image or video file, and affordance 506 enabling users to pause and resume a video. The plurality of interactive affordances may include one or more interactive affordances configured to receive user preferences for image enhancement, including a type of image enhancement. The interactive affordances configured to receive user preferences for image enhancement may include one or more sliders, including slider 502a and slider 502b. Slider 502a may include a moveable element 504a and slider 502b may include a moveable element 504b. The sliders 502a and 502b may be configured to enable a user to input preferences for image enhancement by moving the moveable elements 504a and 504b, respectively, along the sliders 502a and 502b. As described above with reference to FIGS. 1-3 the systems and methods disclosed herein may utilize combined machine learning models (e.g., combined machine learning model 134) to enhance medical images. The combined machine learning models are formed by generating a weighted combination of learned parameters of a plurality of specialized machine learning models (e.g., specialized machine learning models 132). The sliders 502a and 502b may enable users to increase or decrease the contribution of different specialized machine learning models to the combined machine learning model by increasing or decreasing the weight assigned to the respective specialized machine learning models' parameters.

In some examples, the respective affordances configured to receive the user preferences for image enhancement (e.g., slider 502a and slider 502b) may each enable a user to control a contribution of a single specialized machine learning model or multiple specialized machine learning models. In the example shown in FIG. 5A, slider 502a may be configured to control the contribution of multiple specialized machine learning models. As shown in FIG. 5A, the moveable element 504a is located at a first position of slider 502a. The first position may correspond to a relatively lower weight assigned to a specialized machine learning model trained to enhance image softness and a relatively higher weight assigned to a second specialized machine learning model trained to enhance image sharpness. For instance, when the moveable element 504a is in the first position, the weight assigned to the sharpness enhancement model may be 1.0 and the weight assigned to the softness enhancement model may be 0.0. Slider 502a may be configured to control the contribution of a single specialized machine learning model. In the example shown in FIGS. 5A-5C, the specialized machine learning model controlled using slider 502a is trained to enhance image brightness. The moveable element 504b is located at a first position of slider 502b in FIG. 5A, which may correspond to a lower weight assigned to the specialized machine learning model trained to enhance image brightness relative to positions along the slider to the right of the first position in the orientation depicted in FIG. 5A.

The interactive user interface 500 may display a video of a medical procedure, which can be enhanced in real time using the combined machine learning models disclosed herein according to user preferences for image enhancement. In the example depicted in FIG. 5A, the video frame 512a shown has been enhanced using a combined machine learning model formed by generating a weighted combination of the sharpness enhancement, softness enhancement, and brightness enhancement specialized machine learning models controlled by sliders 502a and 502b. The resulting enhanced video frame 512a shown in FIG. 5A has been enhanced primarily based on the learned parameters of the specialized machine learning model trained for sharpness enhancement due to their relatively higher weighting in the combined machine learning model.

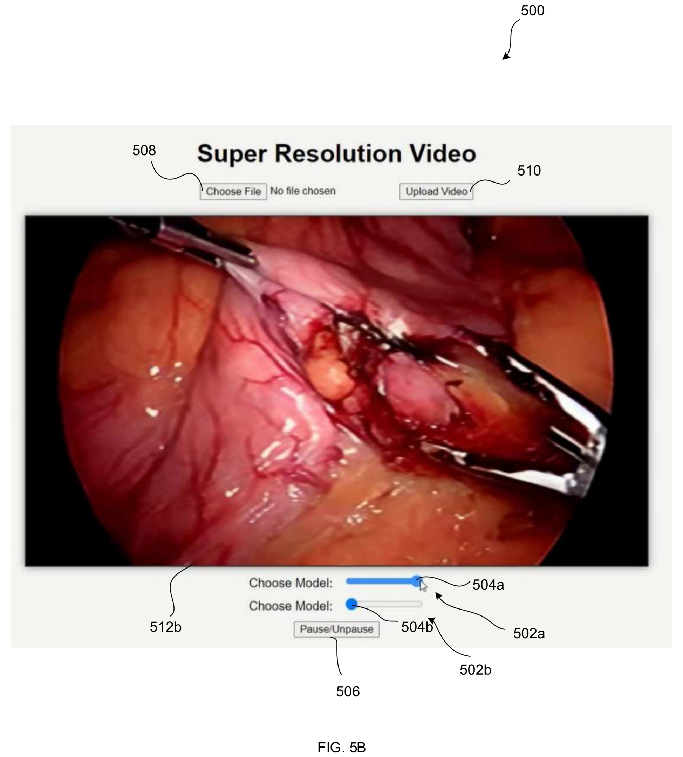

The moveable elements, 504a and 504b, can be moved (e.g., using a drag operation) along the slider to adjust the contribution of one or more of the specialized machine learning models. FIG. 5B illustrates moveable element 504a in a second position of slider 502a (e.g., following a user interaction moving the moveable element 504a from the first position to the second position). At the second position of slider 502a, the contribution (e.g., weight) assigned to the specialized machine learning model trained to enhance image softness is relatively higher than the specialized machine learning model trained to enhance image sharpness. The combined machine learning model is updated by generating another weighted combination of the parameters of the three specialized machine learning models used in the example of FIG. 5B to include the adjusted weight assigned to the specialized machine learning model trained to enhance image sharpness. The video frame 512b shown in FIG. 5B has been enhanced by inputting the frame into the updated combined machine learning model generated using the relatively higher weight assigned to the specialized machine learning model trained to enhance image sharpness. Thus, the resulting video frame 512b depicted in FIG. 5B is relatively softer than the video frame 512a shown in FIG. 5A.

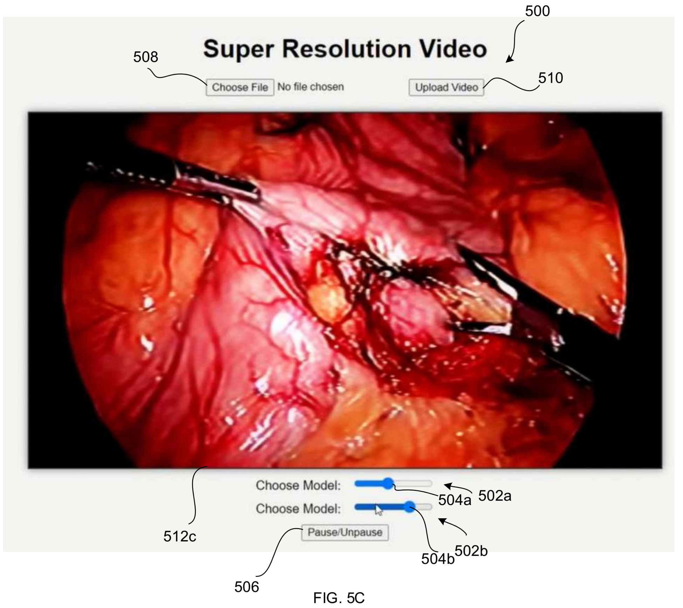

FIG. 5C illustrates moveable element 504a in a third position of slider 502a and moveable element 504b in a second position of slider 502b. At the third position of slider 502a, the contribution (e.g., weight) assigned to the specialized machine learning model trained to enhance image sharpness is relatively higher than the specialized machine learning model trained to enhance image softness. However, at the third position, the weight assigned to the specialized machine learning model trained to enhance image softness is increased relative to the position of the moveable element shown in FIG. 5A. At the second position of the slider 502b, the contribution (e.g., weight) assigned to the specialized machine learning model trained to enhance image brightness is relatively higher than in the first position of slider 502b shown in FIGS. 5A and 5B. The combined machine learning model is updated again by generating yet another weighted combination of the parameters of the three specialized machine learning models used in the example of FIG. 5C. The updated combined machine learning model is generated using the adjusted weight assigned to each specialized machine learning models according to the position of the respective moveable elements 504a and 504b of interactive user interface 500. The video frame 512c shown in FIG. 5B has been enhanced by inputting the frame into the updated combined machine learning model. The combined machine learning model processes the input video frame based on the weighted combination of parameters to generate an enhanced video frame 512c that is relatively brighter and softer than the video frame 512b shown in FIG. 5B.

### Method for Real-Time Adjustment of Combined ML Model Based on User Interaction

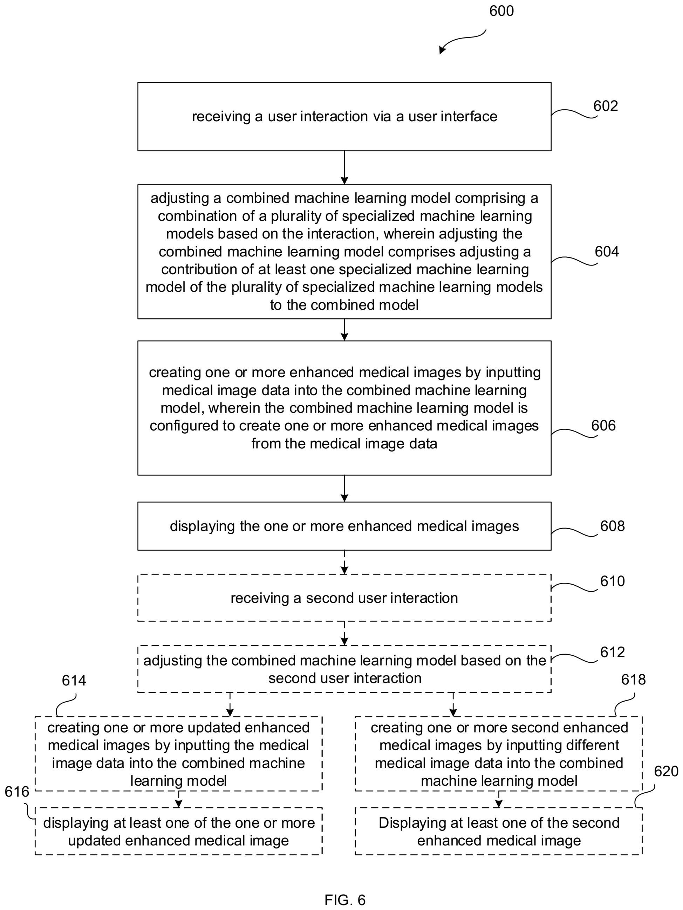

FIG. 6 illustrates an exemplary method 600 for creating one or more enhanced medical images. At block 602, an exemplary system (e.g., system 100) receives a user interaction via a user interface. The interaction may include a user preference for image enhancement. For instance, the interaction may include an indication of a user preference for smoother images, increased brightness, etc. In some examples, the interaction includes a voice command received via an audio sensor. Enabling users to input image enhancement preferences using audio (e.g., voice commands) may be advantageous in that medical professionals may need to maintain control of surgical tools or other equipment during a medical procedure and may not have a free hand to interact with an interactive graphical user interface. In some examples, a graphical user interface (optionally sharing one or more features in common with user interface 500 shown in FIGS. 5A-5C) is provided to enable a user to control image enhancement using interactive affordances of the graphical user interface.

At block 604, the exemplary system adjusts a combined machine learning model (e.g., combined machine learning model 134 or 234) based on the user interaction by adjusting a contribution of at least one specialized machine learning model of a plurality of specialized machine learning models to the combined machine learning model. As discussed with reference to FIG. 2 above, the combined machine learning model is formed by generating a weighted combination of a plurality of specialized machine learning models. The combined machine learning model may be adjustable in real time based on adjustments to a weight assigned to the specialized machine learning models in the weighted combination. For example, the weight assigned to the specialized machine learning models may be applied to the learned parameters of the specialized machine learning models. A user may interact with the system to adjust the weight applied to one or more of the specialized machine learning model parameters. The corresponding parameters of each specialized machine learning model may be combined using multiple-model interpolation to adjust the combined model in real time based on the adjusted weight assigned to one or more of the specialized machine learning models.

Adjusting the weight, and thus the contribution, assigned to any one or more of the specialized machine learning models may in turn adjust the type and/or level (e.g., magnitude) of a given type of image enhancement performed by the combined machine learning model. For instance, the learned parameters (e.g., weights and coefficients) of each of the plurality of specialized machine learning models may be different from the learned parameters of the other specialized machine learning models because each specialized machine learning model is trained for a different type or level of image enhancement. Because the architecture is shared across the specialized machine learning models, the specialized machine learning models can be combined into a single machine learning model using multiple model interpolation. Adjusting the weighting assigned to the parameters for a given specialized machine learning model up or down will, in turn, increase or decrease the contribution of that specialized machine learning model's parameters to the combined machine learning model. Thus, increasing the contribution of a given specialized machine learning model's parameters will in turn increase the type of image enhancement that specialized machine learning model is trained for, and vice versa.

The weight assigned to any one specialized machine learning model may correspond to a percentage of contribution to the combined machine learning model relative to the total contribution of all specialized machine learning models. For instance, parameters of a first specialized machine learning model (e.g., trained for upscaling and contrast enhancement) may be weighted 0.2 and parameters of a second specialized machine learning model (e.g., trained for upscaling and smoothness enhancement) may be weighted 0.8. In such an example, the parameters of the first specialized machine learning model (trained for image upscaling and contrast enhancement) would contribute more to the combined machine learning model than the parameters of the second specialized machine learning model (trained for image upscaling and smoothness enhancement).

The combination of the plurality of specialized machine learning models may include at least two specialized machine learning models selected from the following group: a machine learning model trained for upscaling image resolution and contrast enhancement; a machine learning model trained for upscaling image resolution and brightness enhancement; a machine learning model trained for upscaling image resolution and high dynamic range (HDR) enhancement; a machine learning model trained for upscaling image resolution and sharpness enhancement; a machine learning model trained for upscaling image resolution and softness enhancement; and a machine learning model trained for upscaling image resolution and noise reduction. Adjusting the contribution of at least one specialized machine learning model to the combined machine learning model may include increasing (or decreasing) a contribution of any of the above machine learning models.

In some examples any of the aforementioned machine learning models may not be trained for upscaling (e.g., may only be trained for one of contrast enhancement, brightness enhancement, HDR enhancement, sharpness enhancement, softness enhancement, or noise reduction). Thus, the combination of the plurality of specialized machine learning models may include at least two specialized machine learning models selected from the following group: a machine learning model trained for upscaling image resolution, a machine learning model trained for contrast enhancement; a machine learning model trained for brightness enhancement; a machine learning model trained for high dynamic range (HDR) enhancement; a machine learning model trained for sharpness enhancement; a machine learning model trained for softness enhancement; and a machine learning model trained for noise reduction.

At block 606, the exemplary system creates one or more enhanced medical images by inputting medical image data into the combined machine learning model configured to create one or more enhanced medical images. The medical image data may be a single frame of image data or may include a plurality of frames (e.g., video frames, a video segment, etc.). The system may input individual frames into the combined machine learning model sequentially or may input a plurality of image frames (e.g., a video or video segment) into the combined machine learning model at once (e.g., simultaneously). Thus, the combined machine learning model may receive and enhance a single frame and/or multiple frames with a given input. Creating the one or more enhanced medical images may include generating at least one of: an upscaled medical image, a contrast-enhanced medical image, a high-dynamic-range-enhanced medical image, a brightness-enhanced medical image, a sharpness-enhanced medical image, a softness-enhanced medical image, and a reduced-noise medical image. The combined machine learning model may apply a plurality of image enhancement techniques. For instance, the combined machine learning model may generate an enhanced image that is upscaled, contrast enhanced and sharpened, or an enhanced image that is upscaled, brightened, and softened, or any other combination of the above image enhancement techniques.

At block 608, the exemplary system displays the one or more enhanced medical images (e.g., on a display of the system, such as display 118 of system 100). The display may be included in a medical facility such as in an operating room. Thus, the system may enable medical professionals to view enhanced images (or video) of ongoing surgical or other medical procedures in real time.

At block 610, the system optionally receives a second user interaction via the user interface. The second user interaction may include an updated user preference for image enhancement. For instance, the first interaction may have indicated preference to increase image sharpness. The second interaction may, for instance, indicate a preference to reduce image sharpness (and/or increase image softness). At block 612, the system optionally adjusts the combined machine learning model based on the second user interaction. The system may adjust the combined machine learning model as described with reference to block 604 by adjusting a weight assigned to one or more specialized machine learning models (e.g., to their parameters) based on the second user interaction. For example, the system may reduce the weighting assigned to the sharpness enhancement specialized machine learning model parameters based on the second user interaction. At block 614, the system optionally creates an updated enhanced medical image by inputting the same medical image data back into the adjusted combined machine learning model and generating an updated enhanced medical image using the adjusted configuration of the combined machine learning model. At block 616, the system optionally displays the updated enhanced medical image (e.g., on display 118).

At block 618, the system optionally creates a second enhanced medical image by inputting different medical image data into the combined machine learning model. Thus, the system may enable users to adjust the combined machine learning model, and then new image data may be input into the model to generate a different enhanced image. For example, the system may receive and enhance a first frame of a video stream based on the combined machine learning model configured according to the original user interaction. The combined machine learning model may then be adjusted based on the second user interaction, and a second frame of the video stream may be input into the adjusted combined machine learning model. Thus, the combined machine learning model may be adjustable between respective portions of image data. At block 620, the system optionally displays the second enhanced medical image.

Method 600 is performed, for example, using one or more electronic devices implementing a software platform. Method 600 may be performed using one or more aspects of system 100, for instance, including medical data processing device 116. In some examples, method 600 is performed using a client-server system, and the blocks of method 600 are divided up in any manner between the server and one or more client devices. In some examples, method 600 is performed using a peer-to-peer system, and the blocks of method 600 are divided up in any manner between one or more devices. Thus, while portions of method 600 are described herein as being performed by particular devices, it will be appreciated that method 600 is not so limited. In method 600, some blocks are, optionally, combined, the order of some blocks is, optionally, changed, and some blocks are, optionally, omitted. In some examples, additional steps may be performed in combination with the method 600. Accordingly, the operations as illustrated (and described in greater detail above) are exemplary by nature and, as such, should not be viewed as limiting.

### Method for Configuring Combined ML Model based on Environmental Conditions, Imaging Device, Imaging Modality, Imaging Settings, Medical Procedure, and/or Medical Professional

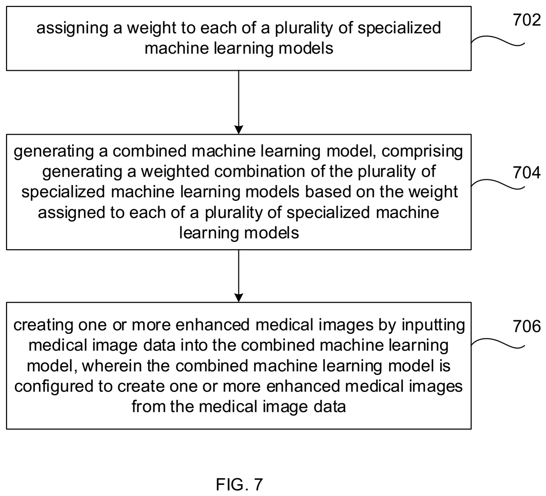

FIG. 7 illustrates an exemplary method 700 for creating one or more enhanced medical images using a combined machine learning model. The specialized machine learning models combined to create the combined machine learning model are assigned a weight that determines their contribution to the combined machine learning model. The weight can be predetermined and/or automatically adjusted based on, for instance, imaging modalities, zoom settings, etc., as described with reference to FIG. 1.

At block 702, an exemplary system performing the method 700 may assign a weight to each of a plurality of specialized machine learning models. A respective weight may be assigned to the plurality of specialized machine learning models based on imaging settings (e.g., zoom settings) and/or environmental conditions, such as lighting, etc. For instance, the contribution of a specialized machine learning model trained for upscaling and brightness enhancement may be increased in a relatively low-lighting setting. The contribution of different specialized machine learning models may additionally, or alternatively, be configured based upon an imaging modality, type of imaging device, resolution of an imaging device, imaging modality, type of surgical procedure, and/or stored preferences of a surgeon or other medical professional. As described above with reference to the method 600, the weight assigned to the specialized machine learning models used to create the combined machine learning model can also be configured and/or adjusted based on user input.

At block 704, the exemplary system may generate a combined machine learning model by generating a weighted combination of the plurality of specialized machine learning models based on the weight assigned to each of a plurality of specialized machine learning models. The combined machine learning model may be generated as described above with reference to FIG. 2. At block 706, the exemplary system may create one or more enhanced medical images by inputting medical image data into the combined machine learning model. The combined machine learning model may process the medical image data to create one or more enhanced medical images from the medical image data. For instance, the combined machine learning model may upscale and enhance contrast and brightness of the input medical image data, upscale and enhance the sharpness and dynamic range of the input medical image data, or otherwise upscale and enhance the input medical image data as described throughout this disclosure.

Method 700 is performed, for example, using one or more electronic devices implementing a software platform. Method 700 may be performed using one or more aspects of system 100, for instance, including medical data processing device 116. In some examples, method 700 is performed using a client-server system, and the blocks of method 700 are divided up in any manner between the server and one or more client devices. In some examples, method 700 is performed using a peer-to-peer system, and the blocks of method 700 are divided up in any manner between one or more devices. Thus, while portions of method 700 are described herein as being performed by particular devices, it will be appreciated that method 700 is not so limited. In method 700, some blocks are, optionally, combined, the order of some blocks is, optionally, changed, and some blocks are, optionally, omitted. In some examples, additional steps may be performed in combination with the method 700. Accordingly, the operations as illustrated (and described in greater detail above) are exemplary by nature and, as such, should not be viewed as limiting.

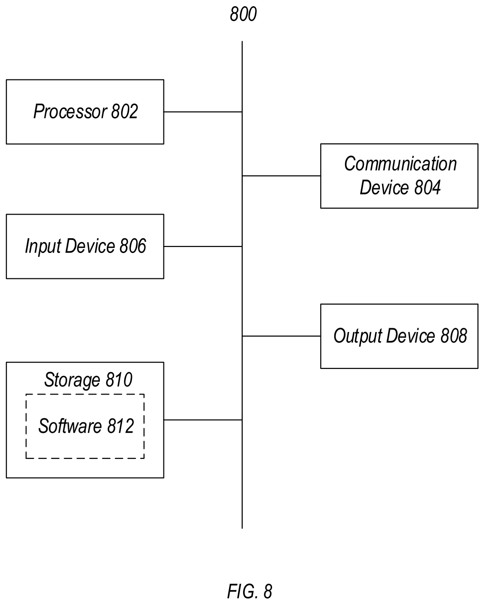

FIG. 8 depicts an exemplary computing device 800, which may be used in accordance with one or more examples of the disclosure. Device 800 can be a host computer connected to a network. Device 800 can be a client computer or a server. As shown in FIG. 8, device 800 can be any suitable type of microprocessor-based device, such as a personal computer, workstation, server, or handheld computing device (portable electronic device) such as a phone or tablet. The device can include, for example, one or more of processors 802, input device 806, output device 808, storage 810, and communication device 804. Input device 806 and output device 808 can generally correspond to those described above and can either be connectable or integrated with the computer.

Input device 806 can be any suitable device that provides input, such as a touch screen, keyboard or keypad, mouse, or voice-recognition device. Output device 808 can be any suitable device that provides output, such as a touch screen, haptics device, or speaker.

Storage 810 can be any suitable device that provides storage, such as an electrical, magnetic, or optical memory, including a RAM, cache, hard drive, or removable storage disk. Communication device 804 can include any suitable device capable of transmitting and receiving signals over a network, such as a network interface chip or device. The components of the computer can be connected in any suitable manner, such as via a physical bus or wirelessly.

Software 812, which can be stored in storage 810 and executed by processor 802, can include, for example, the programming that embodies the functionality of the present disclosure (e.g., as embodied in the devices as described above).

Software 812 can also be stored and/or transported within any non-transitory computer-readable storage medium for use by or in connection with an instruction execution system, apparatus, or device, such as those described above, that can fetch instructions associated with the software from the instruction execution system, apparatus, or device and execute the instructions. In the context of this disclosure, a computer-readable storage medium can be any medium, such as storage 810, that can contain or store programming for use by or in connection with an instruction execution system, apparatus, or device.

Software 812 can also be propagated within any transport medium for use by or in connection with an instruction execution system, apparatus, or device, such as those described above, that can fetch instructions associated with the software from the instruction execution system, apparatus, or device and execute the instructions. In the context of this disclosure, a transport medium can be any medium that can communicate, propagate, or transport programming for use by or in connection with an instruction execution system, apparatus, or device. The transport readable medium can include, but is not limited to, an electronic, magnetic, optical, electromagnetic, or infrared wired or wireless propagation medium.

Device 800 may be connected to a network, which can be any suitable type of interconnected communication system. The network can implement any suitable communications protocol and can be secured by any suitable security protocol. The network can comprise network links of any suitable arrangement that can implement the transmission and reception of network signals, such as wireless network connections, T1 or T3 lines, cable networks, DSL, or telephone lines.

Device 800 can implement any operating system suitable for operating on the network. Software 812 can be written in any suitable programming language, such as C, C++, Java, or Python. In various embodiments, application software embodying the functionality of the present disclosure can be deployed in different configurations, such as in a client/server arrangement or through a Web browser as a Web-based application or Web service, for example.

In the following, numbered embodiments are described. These numbered embodiments can be considered in the light of the above examples, and summarize particular aspects of the invention.

Embodiment 1: A method for creating one or more enhanced medical images, the method comprising: receiving a user interaction via a user interface, the interaction comprising a user preference for image enhancement; adjusting a combined machine learning model comprising a combination of a plurality of specialized machine learning models based on the user interaction, wherein adjusting the combined machine learning model comprises adjusting a contribution of at least one specialized machine learning model of the plurality of specialized machine learning models to the combined model; creating one or more enhanced medical images by inputting medical image data into the combined machine learning model, wherein the combined machine learning model is configured to create one or more enhanced medical images from the medical image data; and displaying the one or more enhanced medical images.

Embodiment 2: The method of embodiment 1, wherein adjusting the contribution of the at least one specialized machine learning model of the combination of specialized machine learning models comprises: adjusting a weight assigned to the at least one specialized machine learning model of the combination of specialized machine learning models; and generating a weighted combination of the plurality of specialized machine learning models including the at least one specialized machine learning model comprising the adjusted weight.

Embodiment 3: The method of embodiment 2, wherein generating the weighted combination of the plurality of specialized machine learning models comprises generating a weighted combination of corresponding learned parameters of each of the plurality of specialized machine learning models.

Embodiment 4: The method of embodiment 1, 2 or 3, wherein each of the plurality of specialized machine learning models comprises an identical model architecture, and wherein: the combined machine learning model comprises the identical model architecture of the plurality of specialized machine learning models.

Embodiment 5: The method of any of embodiments 1-4, wherein the user interface comprises an interactive graphical user interface comprising one or more interactive affordances configured to receive the user preferences for image enhancement.

Embodiment 6: The method of embodiment 5, wherein the one or more interactive affordances comprises a slider, and wherein the user input causes a movement of a moveable element of the slider between a first position of the slider and a second position of the slider.

Embodiment 7: The method of embodiment 5 or 6, wherein adjusting the contribution of the at least one specialized machine learning models comprises: increasing, in real time, a contribution of a first specialized machine learning model based on user manipulation of the one or more interactive affordances, such as on the movement of the moveable element.

Embodiment 8: The method of embodiment 5, 6 or 7, wherein adjusting the contribution of the at least one specialized machine learning models comprises: decreasing, in real time, a contribution of a second specialized machine learning model based on user manipulation of the one or more interactive affordances, such as on the movement of the moveable element.

Embodiment 9: The method of embodiment 6, wherein adjusting the contribution of the at least one specialized machine learning models comprises: increasing, in real time, a contribution of a first specialized machine learning model based on the movement of the moveable element; and decreasing, in real time, a contribution of a second specialized machine learning model based on the movement of the moveable element.

Embodiment 10: The method of any of embodiments 1-9, wherein the user interaction comprises a voice command.

Embodiment 11: The method of any of embodiments 1-10, comprising: receiving a second user interaction via the user interface, the second user interaction comprising a user preference for image enhancement; adjusting the combined machine learning model based on the second user interaction; creating an updated enhanced medical image by inputting the medical image data into the combined machine learning model; and displaying the updated enhanced medical image.

Embodiment 12: The method of any of embodiments 1-11, comprising: receiving a second user interaction via the user interface, the second user interaction comprising a user preference for image enhancement; adjusting the combined machine learning model based on the second user interaction; creating a second enhanced medical image by inputting different medical image data into the combined machine learning model; and displaying the second enhanced medical image.

Embodiment 13: The method of any of embodiments 1-12, wherein the combination of the plurality of specialized machine learning models comprises at least two specialized machine learning models selected from the group comprising: a machine learning model trained for image contrast enhancement; a machine learning model trained for image brightness enhancement; a machine learning model trained for high dynamic range enhancement; a machine learning model trained for image sharpness enhancement; a machine learning model trained for image softness enhancement; and a machine learning model trained for noise reduction.

Embodiment 14: The method of any of embodiments 1-12, wherein the combination of the plurality of specialized machine learning models comprises at least two specialized machine learning models selected from the group comprising: a machine learning model trained for upscaling image resolution and image contrast enhancement; a machine learning model trained for upscaling image resolution and image brightness enhancement; a machine learning model trained for upscaling image resolution and high dynamic range enhancement; a machine learning model trained for upscaling image resolution and image sharpness enhancement; a machine learning model trained for upscaling image resolution and image softness enhancement; and a machine learning model trained for upscaling image resolution and noise reduction.

Embodiment 15: The method of any of embodiments 1-14, wherein each of the specialized machine learning models is a super-resolution convolutional neural network.

Embodiment 16: The method of any of embodiments 1-15, wherein adjusting the contribution of the at least one specialized machine learning model comprises increasing a contribution of a machine learning model trained for image contrast enhancement, and wherein creating the one or more enhanced medical images comprises generating an upscaled and contrast-enhanced medical image using the combined machine learning model.

Embodiment 17: The method of any of embodiments 1-16, wherein adjusting the contribution of the at least one specialized machine learning model comprises increasing a contribution of a machine learning model trained for image high dynamic range enhancement, and wherein creating the one or more enhanced medical images comprises generating an upscaled and high-dynamic-range-enhanced medical image using the combined machine learning model.

Embodiment 18: The method of any of embodiments 1-17, wherein adjusting the contribution of the at least one specialized machine learning model comprises increasing a contribution of a machine learning model trained for brightness enhancement, and wherein creating the one or more enhanced medical images comprises generating an upscaled and brightness-enhanced medical image using the combined machine learning model.

Embodiment 19: The method of any of embodiments 1-18, wherein adjusting the contribution of the at least one specialized machine learning model comprises increasing a contribution of a machine learning model trained for sharpness enhancement, and wherein creating the one or more enhanced medical images comprises generating an upscaled and sharpness-enhanced medical image using the combined machine learning model.

Embodiment 20: The method of any of embodiments 1-19, wherein adjusting the contribution of the at least one specialized machine learning model comprises increasing a contribution of a machine learning model trained for softness enhancement, and wherein creating the one or more enhanced medical images comprises generating an upscaled and softness-enhanced medical image using the combined machine learning model.

Embodiment 21: The method of any of embodiments 1-20, wherein adjusting the contribution of the at least one specialized machine learning model comprises increasing a contribution of a machine learning model trained for noise reduction, and wherein creating the one or more enhanced medical images comprises generating an upscaled and reduced-noise medical image using the combined machine learning model.

Embodiment 22: The method of any of embodiments 1-21, comprising receiving the medical image data from an endoscopic imaging device.

Embodiment 23: A medical imaging system for creating an enhanced medical image, the system comprising one or more processors configured to: receive a user interaction via a user interface, the interaction comprising a user preference for image enhancement; adjust a combined machine learning model comprising a combination of a plurality of specialized machine learning models based on the user interaction, wherein adjusting the combined machine learning model comprises adjusting a contribution of at least one specialized machine learning model of the plurality of specialized machine learning models to the combined model; create one or more enhanced medical images by inputting medical image data into the combined machine learning model, wherein the combined machine learning model is configured to create one or more enhanced medical images from the medical image data; and display the one or more enhanced medical images.

Embodiment 24: A medical imaging system for creating an enhanced medical image, the system comprising one or more processors and a memory storing instructions, which, when executed by the one or more processors cause the system to: receive a user interaction via a user interface, the interaction comprising a user preference for image enhancement; adjust a combined machine learning model comprising a combination of a plurality of specialized machine learning models based on the user interaction, wherein adjusting the combined machine learning model comprises adjusting a contribution of at least one specialized machine learning model of the plurality of specialized machine learning models to the combined model; create one or more enhanced medical images by inputting medical image data into the combined machine learning model, wherein the combined machine learning model is configured to create one or more enhanced medical images from the medical image data; and display the one or more enhanced medical images.

Embodiment 25: The system of embodiment 23 or 24, wherein the system comprises one or more medical imaging devices configured to obtain the medical image data.

Embodiment 26: The system of embodiment 25, wherein the one or more medical imaging devices comprise at least one of an endoscopic imaging device a flex-scope imaging device, fiberscope imaging device, or semirigid scope imaging device, a pan-tilt-zoom (PTZ) camera, an open-field imaging device, and an in-light camera (ILC).

Embodiment 27: The system of any of embodiments 23-26, wherein the user interface comprises at least one of a interactive graphical user interface and an audio sensor.

Embodiment 28: The system any of embodiments 23-27, wherein the plurality of specialized machine learning models comprise an identical model architecture.

Embodiment 29: The system any of embodiments 23-28, wherein the plurality of specialized machine learning models have each been trained using a distinct discriminator model.

Embodiment 30: A computer program product comprising one or more programs for creating an enhanced medical image, the one or more programs comprising instructions, which when executed by one or more processors of an electronic device, cause the electronic device to: receive a user interaction via a user interface, the interaction comprising a user preference for image enhancement; adjust a combined machine learning model comprising a combination of a plurality of specialized machine learning models based on the user interaction, wherein adjusting the combined machine learning model comprises adjusting a contribution of at least one specialized machine learning model of the plurality of specialized machine learning models to the combined model; create one or more enhanced medical images by inputting medical image data into the combined machine learning model, wherein the combined machine learning model is configured to create one or more enhanced medical images from the medical image data; and display the one or more enhanced medical images.

Embodiment 31: A non-transitory computer-readable storage medium storing one or more programs for creating an enhanced medical image, the one or more programs comprising instructions, which when executed by one or more processors of an electronic device, cause the electronic device to: receive a user interaction via a user interface, the interaction comprising a user preference for image enhancement; adjust a combined machine learning model comprising a combination of a plurality of specialized machine learning models based on the user interaction, wherein adjusting the combined machine learning model comprises adjusting a contribution of at least one specialized machine learning model of the plurality of specialized machine learning models to the combined model; create one or more enhanced medical images by inputting medical image data into the combined machine learning model, wherein the combined machine learning model is configured to create one or more enhanced medical images from the medical image data; and display the one or more enhanced medical images.

Embodiment 32: A method for enhancing a medical image, the method comprising: assigning a weight to each of a plurality of specialized machine learning models; generating a combined machine learning model, comprising generating a weighted combination of the plurality of specialized machine learning models based on the weight assigned to each of a plurality of specialized machine learning models; creating one or more enhanced medical images by inputting medical image data into the combined machine learning model, wherein the combined machine learning model is configured to create one or more enhanced medical images from the medical image data.

Embodiment 33: The method of embodiment 32, wherein at least one of the plurality of specialized machine learning models is trained for a different type of image enhancement from a least one other specialized machine learning model of the plurality of specialized machine learning models.

Embodiment 34: The method of embodiment 32 or 33, comprising: causing display of the one or more enhanced medical images.

Embodiment 35: The method of embodiment 32, 33 or 34, comprising: receiving a user input, wherein the user input comprises an indication of a type of image enhancement; adjusting the combined machine learning model based on the user input, wherein adjusting the combined machine learning model comprises adjusting the weight assigned to at least one of the specialized machine learning models; creating a second enhanced medical image by inputting additional medical image data into the adjusted combined machine learning model; and causing display of the second enhanced medical image.

Embodiment 36: The method of any of embodiments 32-35, wherein the weight is assigned to each of the plurality of specialized machine learning models based on a zoom setting of an imaging device that obtained the medical image data.

Embodiment 37: The method of any of embodiments 32-36, wherein the weight is assigned to each of the plurality of specialized machine learning models based on user preferences stored in association with a user profile.

Embodiment 38: The method of any of embodiments 32-37, wherein the weight is assigned to each of the plurality of specialized machine learning models based on any one or more of: a medical procedure, an imaging device, and an imaging modality.

Embodiment 39: A medical imaging system for enhancing a medical image, the system comprising one or more processors configured to: assign a weight to each of a plurality of specialized machine learning models; generate a combined machine learning model, comprising generating a weighted combination of the plurality of specialized machine learning models based on the weight assigned to each of a plurality of specialized machine learning models; create an enhanced medical image by inputting medical image data into the combined machine learning model, wherein the combined machine learning model is configured to create an enhanced medical image from the medical image data.

Embodiment 40: A medical imaging system for enhancing a medical image, the system comprising one or more processors and a memory storing instructions, which, when executed by the one or more processors cause the system to: assign a weight to each of a plurality of specialized machine learning models; generate a combined machine learning model, comprising generating a weighted combination of the plurality of specialized machine learning models based on the weight assigned to each of a plurality of specialized machine learning models; create an enhanced medical image by inputting medical image data into the combined machine learning model, wherein the combined machine learning model is configured to create an enhanced medical image from the medical image data.

Embodiment 41: A computer program product comprising one or more programs for enhancing a medical image, the one or more programs comprising instructions, which when executed by one or more processors of an electronic device, cause the electronic device to: assign a weight to each of a plurality of specialized machine learning models; generate a combined machine learning model, comprising generating a weighted combination of the plurality of specialized machine learning models based on the weight assigned to each of a plurality of specialized machine learning models; create an enhanced medical image by inputting medical image data into the combined machine learning model, wherein the combined machine learning model is configured to create an enhanced medical image from the medical image data.

Embodiment 42: A non-transitory computer-readable storage medium storing one or more programs for enhancing a medical image, the one or more programs comprising instructions, which when executed by one or more processors of an electronic device, cause the electronic device to: assign a weight to each of a plurality of specialized machine learning models; generate a combined machine learning model, comprising generating a weighted combination of the plurality of specialized machine learning models based on the weight assigned to each of a plurality of specialized machine learning models; create an enhanced medical image by inputting medical image data into the combined machine learning model, wherein the combined machine learning model is configured to create an enhanced medical image from the medical image data.

Embodiment 43: A method for creating a combined machine learning model for image enhancement, the method comprising: training a plurality of specialized machine learning models for image enhancement, wherein at least one of the plurality of specialized machine learning models is trained for a different type of image enhancement from at least one other of the plurality of specialized machine learning models; assigning a weight to each of the plurality of specialized machine learning models; and creating the combined machine learning model, comprising generating a weighted combination of the plurality of specialized machine learning models based on the weight assigned to each of a plurality of specialized machine learning models.

Embodiment 44: The method of embodiment 43, wherein at least one of the plurality of specialized machine learning models is trained using a different discriminator model than at least one other of the plurality of specialized machine learning models.

Embodiment 45: The method of embodiment 43 or 44, wherein the weight is assigned based on user preferences received based on a user input, the user input comprising an interaction with at least one of the one or more interactive affordances of a graphical user interface.

Embodiment 46: The method of embodiment 43, 44 or 45, wherein generating the weighted combination of the plurality of specialized machine learning models comprises generating a weighted combination of corresponding learned parameters of the plurality of specialized machine learning models.

Embodiment 47: A system for creating a combined machine learning model for image enhancement, the system comprising one or more processors configured to: train a plurality of specialized machine learning models for respective types of image enhancement; assign a weight to each of the plurality of specialized machine learning models; and create the combined machine learning model, comprising generating a weighted combination of the plurality of specialized machine learning models based on the weight assigned to each of a plurality of specialized machine learning models.

Embodiment 48: A system for creating a combined machine learning model for image enhancement, the system comprising one or more processors and a memory storing instructions, which, when executed by the one or more processors cause the system to: train a plurality of specialized machine learning models for respective types of image enhancement; assign a weight to each of the plurality of specialized machine learning models; and create the combined machine learning model, comprising generating a weighted combination of the plurality of specialized machine learning models based on the weight assigned to each of a plurality of specialized machine learning models.

Embodiment 49: A computer program product comprising one or more programs for creating a combined machine learning model for image enhancement, the one or more programs comprising instructions, which when executed by one or more processors of an electronic device, cause the electronic device to: train a plurality of specialized machine learning models for respective types of image enhancement; assign a weight to each of the plurality of specialized machine learning models; and create the combined machine learning model, comprising generating a weighted combination of the plurality of specialized machine learning models based on the weight assigned to each of a plurality of specialized machine learning models.

Embodiment 50: A non-transitory computer-readable storage medium storing one or more programs for creating a combined machine learning model for image enhancement, the one or more programs comprising instructions, which when executed by one or more processors of an electronic device, cause the electronic device to: train a plurality of specialized machine learning models for respective types of image enhancement; assign a weight to each of the plurality of specialized machine learning models; and create the combined machine learning model, comprising generating a weighted combination of the plurality of specialized machine learning models based on the weight assigned to each of a plurality of specialized machine learning models.

The foregoing description, for the purpose of explanation, has been described with reference to specific embodiments. However, the illustrative discussions above are not intended to be exhaustive or to limit the invention to the precise forms disclosed. Many modifications and variations are possible in view of the above teachings. The embodiments were chosen and described in order to best explain the principles of the techniques and their practical applications. Others skilled in the art are thereby enabled to best utilize the techniques and various embodiments with various modifications as are suited to the particular use contemplated.

Although the disclosure and examples have been fully described with reference to the accompanying figures, it is to be noted that various changes and modifications will become apparent to those skilled in the art. Such changes and modifications are to be understood as being included within the scope of the disclosure and examples as defined by the claims. Finally, the entire disclosure of the patents and publications referred to in this application are hereby incorporated herein by reference.

For the purpose of clarity and a concise description, features are described herein as part of the same or separate examples; however, it will be appreciated that the scope of the disclosure includes examples having combinations of all or some of the features described.

**Claims**

1. A method for creating one or more enhanced medical images, the method comprising:

receiving a user interaction via a user interface, the interaction comprising a user preference for image enhancement;

adjusting a combined machine learning model comprising a combination of a plurality of specialized machine learning models based on the user interaction, wherein adjusting the combined machine learning model comprises adjusting a contribution of at least one specialized machine learning model of the plurality of specialized machine learning models to the combined model;

creating one or more enhanced medical images by inputting medical image data into the combined machine learning model, wherein the combined machine learning model is configured to create one or more enhanced medical images from the medical image data; and

displaying the one or more enhanced medical images.

2. The method of claim 1, wherein adjusting the contribution of the at least one specialized machine learning model of the combination of specialized machine learning models comprises:

adjusting a weight assigned to the at least one specialized machine learning model of the combination of specialized machine learning models; and

generating a weighted combination of the plurality of specialized machine learning models including the at least one specialized machine learning model comprising the adjusted weight, such as by generating a weighted combination of corresponding learned parameters of each of the plurality of specialized machine learning models.

3. The method of claim 1 or 2, wherein each of the plurality of specialized machine learning models comprises an identical model architecture, and wherein:

the combined machine learning model comprises the identical model architecture of the plurality of specialized machine learning models.

4. The method of any one of the preceding claims, wherein the user interface comprises an interactive graphical user interface comprising one or more interactive affordances configured to receive the user preferences for image enhancement, and/or an audio sensor for receiving voice commands.

5. The method of claim 4, wherein the one or more interactive affordances comprises a slider, and wherein the user input causes a movement of a moveable element of the slider between a first position of the slider and a second position of the slider.

6. The method of claim 4 or 5, wherein adjusting the contribution of the at least one specialized machine learning model comprises:

increasing, in real time, a contribution of a first specialized machine learning model based on user manipulation of the one or more interactive affordances, such as on the movement of the moveable element; and/or

decreasing, in real time, a contribution of a second specialized machine learning model based user manipulation of the one or more interactive affordances, such as on the movement of the moveable element.

7. The method of any one of the preceding claims, comprising:

receiving a second user interaction via the user interface, the second user interaction comprising a user preference for image enhancement;

adjusting the combined machine learning model based on the second user interaction;

creating an updated enhanced medical image by inputting the medical image data into the combined machine learning model and/or creating a second enhanced medical image by inputting different medical image data into the combined machine learning model; and

displaying the updated enhanced medical image.

8. The method of any one of the preceding claims, wherein the combination of the plurality of specialized machine learning models comprises at least two specialized machine learning models selected from the group comprising:

a machine learning model trained for upscaling image resolution and image contrast enhancement;

a machine learning model trained for upscaling image resolution and image brightness enhancement;

a machine learning model trained for upscaling image resolution and high dynamic range enhancement;

a machine learning model trained for upscaling image resolution and image sharpness enhancement;

a machine learning model trained for upscaling image resolution and image softness enhancement; and

a machine learning model trained for upscaling image resolution and noise reduction.

9. The method of any one of the preceding claims, wherein each of the specialized machine learning models is a super-resolution convolutional neural network.

10. The method of any one of the preceding claims, wherein adjusting the contribution of the at least one specialized machine learning model comprises one or more of:

A) increasing a contribution of a machine learning model trained for image contrast enhancement, for generating an upscaled and contrast-enhanced medical image using the combined machine learning model,
B) increasing a contribution of a machine learning model trained for image high dynamic range enhancement, for generating an upscaled and high-dynamic-range-enhanced medical image using the combined machine learning model,
C) increasing a contribution of a machine learning model trained for brightness enhancement, for generating an upscaled and brightness-enhanced medical image using the combined machine learning model,
D) increasing a contribution of a machine learning model trained for sharpness enhancement, for generating an upscaled and sharpness-enhanced medical image using the combined machine learning model,
E) increasing a contribution of a machine learning model trained for softness enhancement, for generating an upscaled and softness-enhanced medical image using the combined machine learning model, and
F) increasing a contribution of a machine learning model trained for noise reduction, for generating an upscaled and reduced-noise medical image using the combined machine learning model.

11. A medical imaging system for creating an enhanced medical image, the system comprising one or more processors configured to:

receive a user interaction via a user interface, the interaction comprising a user preference for image enhancement;
adjust a combined machine learning model comprising a combination of a plurality of specialized machine learning models based on the user interaction, wherein adjusting the combined machine learning model comprises adjusting a contribution of at least one specialized machine learning model of the plurality of specialized machine learning models to the combined model;
create one or more enhanced medical images by inputting medical image data into the combined machine learning model, wherein the combined machine learning model is configured to create one or more enhanced medical images from the medical image data; and
display the one or more enhanced medical images.

12. The system of claim 11, wherein the system comprises one or more medical imaging devices configured to obtain the medical image data, such as at least one of an endoscopic imaging device a flex-scope imaging device, fiberscope imaging device, or semirigid scope imaging device, a pan-tilt-zoom (PTZ) camera, an open-field imaging device, and an in-light camera (ILC).

13. The system of claim 11 or 12, wherein the user interface comprises at least one of a interactive graphical user interface and an audio sensor.

14. The system of claim 11, 12 or 13, wherein the plurality of specialized machine learning models comprise an identical model architecture, and/or have each been trained using a distinct discriminator model.

15. A computer program product comprising, or a non-transitory computer-readable storage medium storing, one or more programs for creating an enhanced medical image, the one or more programs comprising instructions, which when executed by one or more processors of an electronic device, cause the electronic device to:

receive a user interaction via a user interface, the interaction comprising a user preference for image enhancement;
adjust a combined machine learning model comprising a combination of a plurality of specialized machine learning models based on the user interaction, wherein adjusting the combined machine learning model comprises adjusting a contribution of at least one specialized machine learning model of the plurality of specialized machine learning models to the combined model;
create one or more enhanced medical images by inputting medical image data into the combined machine learning model, wherein the combined machine learning model is configured to create one or more enhanced medical images from the medical image data; and
display the one or more enhanced medical images.

FIG. 1

232

232

Mi

. . .

Mn

Combine learned
parameters

234

Combined Machine Learning
Model

FIG. 2

FIG. 3

FIG. 4

FIG. 5A

FIG. 5B

FIG. 5C

600

receiving a user interaction via a user interface — 602

adjusting a combined machine learning model comprising a combination of a plurality of specialized machine learning models based on the interaction, wherein adjusting the combined machine learning model comprises adjusting a contribution of at least one specialized machine learning model of the plurality of specialized machine learning models to the combined model — 604

creating one or more enhanced medical images by inputting medical image data into the combined machine learning model, wherein the combined machine learning model is configured to create one or more enhanced medical images from the medical image data — 606

displaying the one or more enhanced medical images — 608

receiving a second user interaction — 610

adjusting the combined machine learning model based on the second user interaction — 612

614

creating one or more updated enhanced medical images by inputting the medical image data into the combined machine learning model

616

displaying at least one of the one or more updated enhanced medical image

618

creating one or more second enhanced medical images by inputting different medical image data into the combined machine learning model

620

Displaying at least one of the second enhanced medical image

FIG. 6

| assigning a weight to each of a plurality of specialized machine learning models | 702 |

| generating a combined machine learning model, comprising generating a weighted combination of the plurality of specialized machine learning models based on the weight assigned to each of a plurality of specialized machine learning models | 704 |

| creating one or more enhanced medical images by inputting medical image data into the combined machine learning model, wherein the combined machine learning model is configured to create one or more enhanced medical images from the medical image data | 706 |

FIG. 7

*800*

```
┌─────────────────┐
│                 │
│  Processor 802  │────┐
│                 │    │
└─────────────────┘    │         ┌──────────────────┐
                       │         │                  │
                       ├─────────│  Communication   │
                       │         │   Device 804     │
┌─────────────────┐    │         │                  │
│                 │    │         └──────────────────┘
│ Input Device 806│────┤
│                 │    │
└─────────────────┘    │         ┌──────────────────┐
                       │         │                  │
                       ├─────────│ Output Device 808│
                       │         │                  │
┌─────────────────┐    │         └──────────────────┘
│ Storage 810     │    │
│ ┌─────────────┐ │    │
│ │Software 812 │ │────┘
│ └─────────────┘ │
└─────────────────┘
```

*FIG. 8*

Europäisches Patentamt
European Patent Office
Office européen des brevets

# EUROPEAN SEARCH REPORT

**Application Number**

EP 25 22 7437

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | JUNG WOOCHAN ET AL: "Image Quality Enhancement via Machine Learning: A Unified Approach to Super-Resolution, Denoising, and Low-Density Enhancement", JOURNAL OF STUDENT RESEARCH, vol. 12, no. 4, 30 November 2023 (2023-11-30), XP093376408, ISSN: 2167-1907, DOI: 10.47611/jsrhs.v12i4.5541 * the whole document * | 1-15 | INV. G06T5/60 |
| A | QIYUAN LIANG ET AL: "Image Denoising with Control over Deep Network Hallucination", ARXIV.ORG CORNELL UNIVERSITY LIBRARY, 201 OLIN LIBRARY CORNELL UNIVERSITY ITHACA, NY 14853, 2 January 2022 (2022-01-02), XP091131178, * the whole document * | 1-15 | |

-----

-----

**TECHNICAL FIELDS SEARCHED (IPC)**

G06T

The present search report has been drawn up for all claims

| Place of search | Date of completion of the search | Examiner |
|---|---|---|
| Munich | 13 March 2026 | Yilmaz, Özgün |

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63740863

**Non-patent literature cited in the description**

- **CHRISTIAN LEDIG et al.** Photo-Realistic Single Image Super-Resolution Using a Generative Adversarial Network. *CVPR*, 2017, https://doi.org/10.48550/arXiv.1609.04802

- Photo-Realistic Single Image Super-Resolution Using a Generative Adversarial Network. *CVPR*, 2017, https://doi.org/10.48550/arXiv.1609.04802